(19) Europäisches Patentamt
European Patent Office
Office européen des brevets

(11) Veröffentlichungsnummer: **0 095 076**
**B1**

(12) # EUROPÄISCHE PATENTSCHRIFT

(45) Veröffentlichungstag der Patentschrift: **01.04.87**

(51) Int. Cl.⁴: **C 07 D 498/10**, C 08 K 5/35 // (C07D498/10, 263:00, 221:00)

(21) Anmeldenummer: **83104471.4**

(22) Anmeldetag: **06.05.83**

(54) **Polyalkyldiazaspirodecanylessigsäurederivate, Verfahren zu ihrer Herstellung und ihre Verwendung als Lichtschutzmittel für organische Polymere.**

(30) Priorität: **12.05.82 DE 3217734**

(43) Veröffentlichungstag der Anmeldung: **30.11.83 Patentblatt 83/48**

(45) Bekanntmachung des Hinweises auf die Patenterteilung: **01.04.87 Patentblatt 87/14**

(84) Benannte Vertragsstaaten: **AT BE CH DE FR GB IT LI NL SE**

(56) Entgegenhaltungen:
**EP - A - 0 001 207**
**EP - A - 0 008 084**
**EP - A - 0 022 997**
**EP - A - 0 025 867**
**DE - A - 2 227 689**
**GB - A - 2 089 800**

(73) Patentinhaber: **HOECHST AKTIENGESELLSCHAFT, Postfach 80 03 20, D-6230 Frankfurt am Main 80 (DE)**

(72) Erfinder: **Wiezer, Hartmut, Dr., Am Stocket 18, D-8901 Lützelburg (DE)**

**EP 0 095 076 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus der Literatur sind zahlreiche unsubstituierte und substituierte Diazaspirodecane vom Oxazolidinontyp bekannt. So beschreiben die DE-PSS 2 606 026 und 2 634 957 am Oxazolidinonstickstoff unsubstituierte Verbindungen aus dieser Klasse, die sich durch eine recht gute Stabilisatorwirksamkeit auszeichnen, jedoch besitzen sie noch z.T. erhebliche Nachteile, insbesondere hinsichtlich der Flüchtigkeit und auch der in manchen Kunststoffen mangelhaften Löslichkeit. Dies hat zur Folge, dass diese Produkte nicht universell, d.h. nicht mit gleichem Erfolg auf mehreren Gebieten, wie z.B. dem Lacksektor und dem Kunststoffsektor, einsetzbar sind.

Es hat nun nicht an Versuchen gefehlt, diese Nachteile durch Vergrösserung des Moleküls oder Substitution an der polaren NH-Gruppe durch Alkylreste zu beheben. So sind in der EP-B-17 617 am Oxazolidinonstickstoff substituierte Diazaspirodecane beschrieben, die jedoch besonders wegen ihrer zu hohen Flüchtigkeit technisch nicht befriedigen. Die aus der DE-OS 2 933 732 bekannten N-Alkyldiazaspirodecane besitzen demgegenüber z.T. eine beachtlich niedrige Flüchtigkeit, lassen sich aber nur schwierig und in ungenügender Ausbeute herstellen.

Der Erfindung lag nun die Aufgabe zugrunde, Diazaspirodecanderivate bereitzustellen, die gut wirksam, wenig flüchtig und mit den zu stabilisierenden Polymeren gut verträglich sind, wobei als weiteres Kriterium auf hohe Ausbeuten Wert zu legen war.

Es wurde nun gefunden, dass relativ leicht und mit guten Ausbeuten zugängliche Diazaspirodecanylessigsäurederivate überraschenderweise diese Anforderungen in hohem Masse erfüllen.

Die erfindungsgemässen Verbindungen entsprechen der allgemeinen Formel I

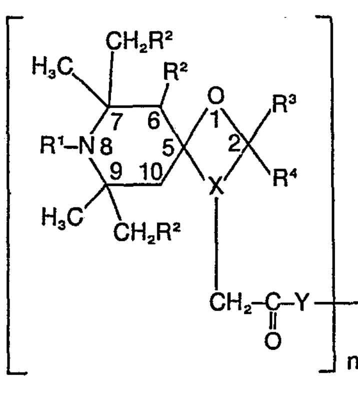 (I),

in welcher

X die Bedeutung einer Gruppe der Formel II oder III hat,

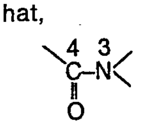 (II) 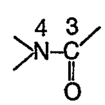 (III)

wobei die Indices 3 und 4 die Ringposition im Diazaspirodecansystem angeben und die freie Valenz des Stickstoffatoms 3 bzw. 4 die Verknüpfung mit dem Essigsäurerest bewirkt.

$R^1$ bedeutet Wasserstoff, Sauerstoff oder $C_1$- bis $C_{12}$-Alkyl, vorzugsweise Wasserstoff, Sauerstoff oder $C_1$- bis $C_4$-Alkyl und insbesondere Wasserstoff,

$R^2$ Wasserstoff oder eine $C_1$- bis $C_5$-Alkylgruppe, vorzugsweise Wasserstoff oder eine Methylgruppe und insbesondere Wasserstoff.

$R^3$ steht für Wasserstoff, $C_1$- bis $C_{30}$-, vorzugsweise $C_1$- bis $C_{18}$- und insbesondere $C_1$- bis $C_5$-Alkyl, für eine Phenyl- oder Naphthylgruppe, die bis zu 2fach durch Chlor oder $C_1$- bis $C_4$-Alkyl-substituiert sein kann, vorzugsweise für Phenyl, oder für eine $C_7$- bis $C_{12}$-Phenylalkylgruppe, die durch einen $C_1$- bis $C_4$-Alkylrest substituiert sein kann, vorzugsweise für eine Benzylgruppe.

$R^4$ hat die Bedeutung von Wasserstoff, von $C_1$- bis $C_{30}$-, vorzugsweise $C_1$- bis $C_{18}$- und insbesondere $C_1$- bis $C_{13}$-Alkyl, von Phenyl oder Naphthyl, das bis zu 2fach durch Chlor oder $C_1$- bis $C_4$-Alkyl substituiert sein kann, vorzugsweise von Phenyl oder von $C_7$- bis $C_{12}$-Phenylalkyl, das durch einen $C_1$- bis $C_4$-Alkylrest substituiert sein kann, vorzugsweise jedoch von Benzyl.

$R^3$ und $R^4$ können zusammen mit dem sie bindenden Kohlenstoffatom auch für eine $C_5$- bis $C_{18}$-, vorzugsweise $C_5$- bis $C_{12}$-Cycloalkylgruppe, die durch bis zu vier $C_1$- bis $C_4$-Alkylgruppen, vorzugsweise Methylgruppen substituiert sein kann, oder für eine Gruppe der Formel

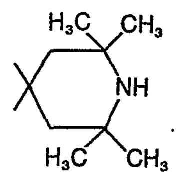

stehen.

n bedeutet eine ganze Zahl von 1 bis 6, vorzugsweise 1 bis 4 und insbesondere 1 bis 3.

$R^5$ hat bei n = 1 die Bedeutung von Wasserstoff, von $C_1$- bis $C_{18}$-, vorzugsweise $C_1$- bis $C_{12}$-Alkyl, von $C_7$- bis $C_9$-Phenylalkyl, das bis zu 2fach durch $C_1$- bis $C_4$-Alkyl substituiert sein kann, oder von $C_3$- bis $C_{12}$-ALkenyl, oder steht für eine 2,2,6,6-Tetramethyl-4-piperidinylgruppe.

Bei n = 2 ist $R^5$ eine $C_2$- bis $C_{30}$-, vorzugsweise $C_2$- bis $C_{18}$- und insbesondere $C_2$- bis $C_6$-Alkylengruppe, oder eine $C_4$- bis $C_8$-Alkenylengruppe, oder eine $C_2$- bis $C_{12}$-Bis-(propyloxi)-alkylengruppe, oder ein Mono-, Di- oder Tricycloalkylen mit 6 bis 18, vorzugsweise 6 bis 12 C-Atomen, das durch bis zu vier Methylgruppen substituert sein kann, vorzugsweise jedoch unsubstituiert ist, wobei bei dem erstgenannten zwei C-Atome durch N-Atome, welche Propylengruppen tragen können, ersetzt sein können, oder $C_6$- bis $C_{18}$-Arylen, vorzugsweise Phenylen, oder $C_7$- bis $C_{18}$-Aralkylen.

Bei n = 3 ist $R^5$ ein $C_3$- bis $C_6$-Alkantriylrest, oder ein Rest der Formel

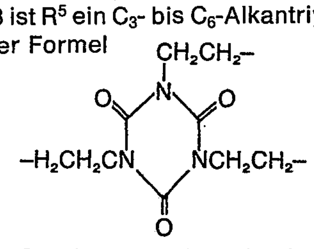

oder ein Di-$C_2$- bis $C_4$-Alkylen-triaminrest.

Bei n = 4 bis 6 ist $R^5$ ein $C_4$- bis $C_{10}$-Alkan-n-ylrest, der eine Ethergruppe enthalten kann, oder ein Tri- bis Penta-$C_2$- bis $C_4$-, vorzugsweise $-C_2$-Alkylen-tetra-bis-hexamin-Rest.

Y steht für –O– oder $-\overset{R^6}{\overset{|}{N}}-$, wobei $R^6$ eine der unter $R^5$ bei n = 1 angegebenen Bedeutungen hat.

Beispiele für erfindungsgemässe Polyalkyldiazaspirodecanylessigsäurederivate sind:

1. 2,2,7,7,9,9-Hexamethyl-1-oxa-3,8-diaza-4-oxo-spiro-[4,5]-decan-3-yl-essigsäureethylester
2. 2,2,7,7,9,9-Hexamethyl-1-oxa-4,8-diaza-3-oxo-spiro-[4,5]-decan-4-yl-essigsäureethylester
3. 2,2,7,7,9,9,-Hexamethyl-1-oxa-3,8-diaza-4-oxo-spiro-[4,5]-decan-3-yl-essigsäure-(2,2,6,6-tetramethyl-4-piperidinyl)-ester
4. Ethan-bis-(2,2,7,7,9,9-hexamethyl-1-oxa-4,8-diaza-3-oxo-spiro-[4,5]-decan-4-yl)-essigsäureamid
5. 7,7,9,9-Tetramethyl-2,2-diethyl-1-oxa-3,8-diaza-4-oxo-spiro-[4,5]-decan-3-yl-essigsäureethylester
6. 1,4-Buten-bis-(7,7,9,9-tetramethyl-2,2-diethyl-1-oxa-3,8-diaza-4-oxo-spiro[4,5]-decan-3-yl)-essigsäureester
7. 1,6-Hexan-bis-(7,7,9,9-tetramethyl-2,2-diethyl-1-oxa-3,8-diaza-4-oxo-spiro-[4,5]-decan-3-yl)-essigsäureester
8. 1,6-Hexan-bis-(7,7,9,9-tetramethyl-2,2-diethyl-1-oxa-3,8-diaza-4-oxo-spiro-[4,5]-decan-3-yl)-essigsäureamid
9. 7,7,9,9-Tetramethyl-2,2-diethyl-1-oxa-3,8-diaza-4-oxo-spiro-[4,5]-decan-3-yl-essigsäure-(2,2,6,6-tetramethyl-4-piperidinyl)-ester
10. 7,7,9,9-Tetramethyl-2-ethyl-2-pentyl-1-oxa-3,8-diaza-4-oxo-spiro-[4,5]-decan-3-yl-essigsäuremethylester
11. 2,7,7,9,9-Pentamethyl-2-propyl-1-oxa-3,8-diaza-4-oxo-spiro-[4,5]-decan-3-yl-essigsäuremethylester
12. 2,7,7,9,9-Pentamethyl-2-undecyl-1-oxa-3,8-diaza-4-oxo-spiro-[4,5]-decan-3-yl-essigsäuremethylester
13. 2,2,4,4-Tetramethyl-10-tert.-butyl-7-oxa-3,13-diaza-14-oxo-dispiro-[5.1.4.2]-tetradecan-13-yl-essigsäuremethylester
14. 2,7,7,9,9-Pentamethyl-2-undecyl-1-oxo-3,8-diaza-4-oxo-spiro-[4,5]-decan-3-yl-essigsäureamid
15. Bis-(2,2,4,4-tetramethyl-10-tert.-butyl-7-oxa-3,13-diaza-14-oxo-dispiro-[5.1.4.2]-tetradecan-13-yl)-essigsäureglykolester
16. Bis-(2,7,7,9,9-pentamethyl-2-benzyl-1-oxa-3,8-diaza-4-oxo-spiro-[4,5]-decan-3-yl-essigsäureglykolester
17. 7,7,9,9,-Tetramethyl-2,2-dibenzyl-1-oxa-3,8-diaza-4-oxo-spiro-[4,5]-decan-3-yl)-essigsäureamid
18. 2,7,7,9,9-Pentamethyl-2-benzyl-1-oxa-3,8-diaza-4-oxo-spiro-[4,5]-decan-3-yl)-essigsäureamid
19. 7,7,9,9-Tetramethyl-2,2-dibenzyl-1-oxa-3,8-diaza-4-oxo-spiro-[4,5]-decan-3-yl)-essigsäureethylester
20. 2,2,4,4-Tetramethyl-7-oxa-3,20-diaza-21-oxo-dispiro-[5.1.11.2]-heneicosan-20-yl-essigsäureethylester
21. 2,2,4,4-Tetramethyl-7-oxa-3,20-diaza-21-oxo-dispiro-[5.1.11.2]-heneicosan-20-yl-essigsäure-(2,2,6,6-tetramethyl-4-piperidinyl)-ester
22. 2,2,4,4-Tetramethyl-7-oxa-3,20-diaza-21-oxo-dispiro-[5.1.11.2]-heneicosan-20-yl-essigsäurehexylester
23. 2,2,4,4-Tetramethyl-7-oxa-3,20-diaza-21-oxo-dispiro-[5.1.11.2]-heneicosan-20-yl-essigsäure-2-ethyl-hexyl-ester
24. Tris-(2,2,4,4-tetramethyl-7-oxa-3,20-diaza-21-oxo-dispiro-[5.1.11.2]-heneicosan-20-yl)-essigsäurepropantriylester
25. Bis-(2,2,4,4-tetramethyl-7-oxa-3,20-diaza-21-oxo-dispiro-[5.1.11.2]-heneicosan-20-yl)-essigsäurehexandiylester
26. 1,6-Hexan-bis-(2,2,4,4-tetramethyl-7-oxa-3,20-diaza-21-oxo-dispiro-[5.1.11.2]-heneicosan-20-yl-essigsäureamid
27. N,N'-3(4),8(9)-bis-methylen-tricyclo-$[5.2.1.0^{2,6}]$-decan-bis-(2,2,4,4-tetramethyl-7-oxa-3,20-diaza-21-oxo-dispiro-[5.1.11.2]-heneicosan-20-yl)-essigsäureamid
28. Tris-(2,2,7,7,9,9-hexamethyl-1-oxa-4,8-diaza-3-oxo-spiro-[4,5]-decan-4-yl)-essigsäurepropantriylester
29. Tris-(1,3,5-tri-ethylen-triazin-2,4,6-trion)-(2,2,7,7,9,9-hexamethyl-1-oxa-3,8-diaza-4-oxo-spiro-[4,5]-decan-3-yl)-essigsäureester
30. N,N'-3(4),8(9)-bis-methylen-tricyclo-$[5.2.1.0^{2,6}]$-decan-bis-(2,2,7,7,9,9-hexamethyl-1-oxa-3,8-diaza-4-oxo-spiro-[4,5]-decan-3-yl)-essigsäureamid
31. 2,2,4,4-Tetramethyl-7-oxa-3,13-diaza-14-oxo-dispiro-[5.1.4.2]-tetradecan-3-yl-essigsäureethylester

Die erfindungsgemässen Diazaspirodecanylessigsäurederivate werden gemäss dem nachstehend skizzierten Schema aus den Verbindungen der DE-PSS 2 606 026, 2 634 957 und 2 834 962 IV durch Umsetzen mit Halogenessigsäurederivaten V entweder in einer einzigen Verfahrensstufe nach Weg A oder aus nach Weg A gewonnenen Methyl- oder Ethylestern Ia durch Umesterung mit alkoholischen Verbindungen oder Amidierung mit Aminogruppen tragenden Verbindungen VI entsprechend Weg B erhalten.

In den Formeln des Reaktionsschemas haben $R^1$, $R^2$, $R^3$, $R^4$, $R^5$, n, X und Y die oben angegebenen Bedeutungen; Hal bedeutet Chlor oder Brom.

Bei der Herstellung geht man im einzelnen so vor, dass man zunächst aus den Diazaspirodecanen der Formel IV durch Umsetzen mit Alkalimetallhydriden, -alkoholaten oder -hydroxiden die entsprechenden Salze herstellt, welche sodann gemäss Weg A bei Temperaturen von 100 bis 200, vorzugsweise 120 bis 200 und insbesondere 140 bis 200 °C in inerten organischen Lösungsmitteln wie z.B. Toluol, Xylol oder Dimethylformamid

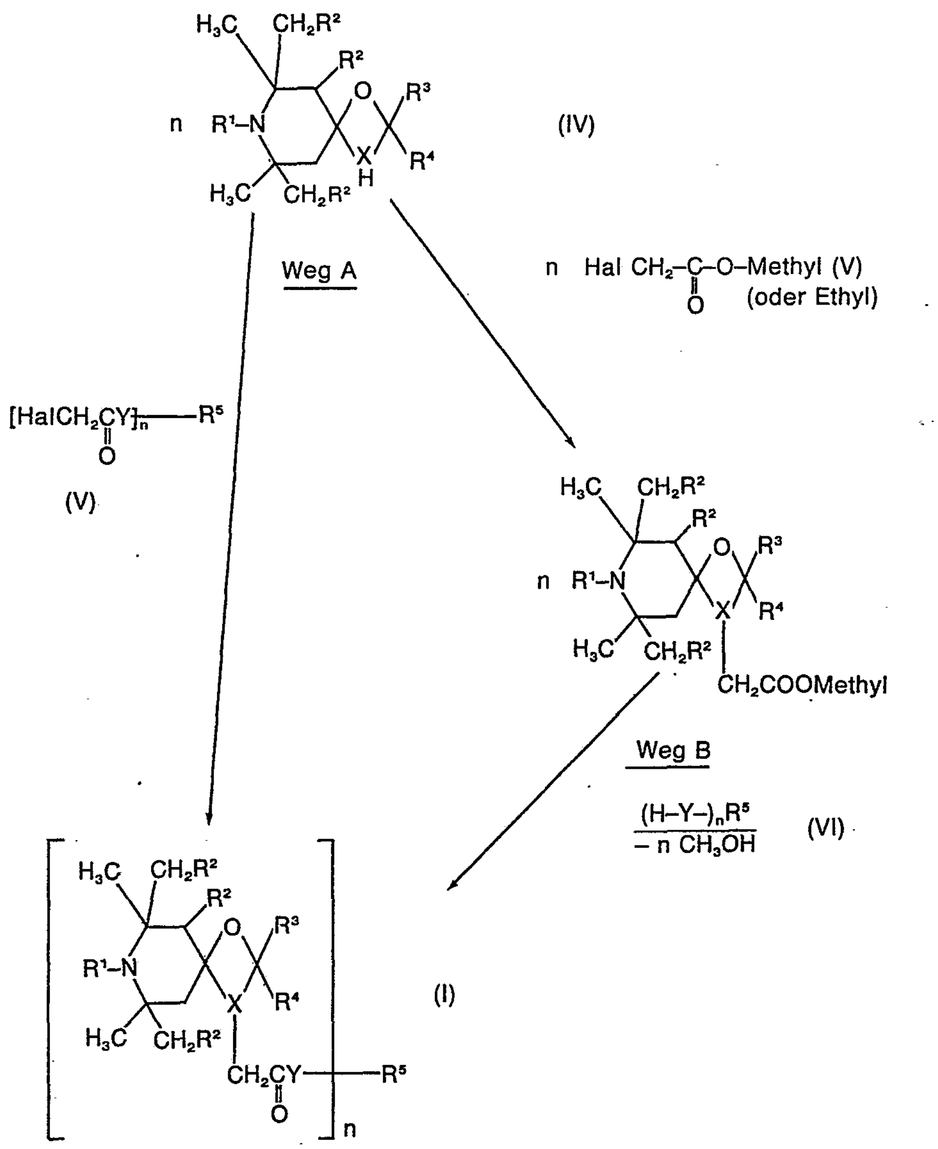


(IV)

(I a)

(I)

drucklos oder unter Druck mit Halogenessigsäurederivaten V zu den gewünschten Verbindungen der Formel I umgesetzt werden. Es ist auch möglich und in vielen Fällen vorteilhaft, die nach Weg A erhältlichen, einen Teil der Erfindung darstellenden Methyl- oder Ethylester Ia nach Weg B in andere erfindungsgemässe Produkte durch Umesterung oder Amidierung überzuführen. Die letzteren Reaktionen werden bei den vorgenannten Reaktionstemperaturen vorzugsweise in aromatischen Kohlenwasserstoffen als Lösungsmittel unter Zusatz von üblichen Katalysatoren wie z.B. $LiNH_2$, $NaOCH_3$, oder $Ti(O\text{-}i\text{-}Prop)_4$ durchgeführt.

Es war überraschend und nicht vorhersehbar, dass die erfindungsgemässen Polyalkyldiazaspirodecanylessigsäuren deutlich weniger flüchtig sein würden als die entsprechenden Alkylderivate der EP-B-17 617 bzw. der DE-OS 2 933 732. Vielmehr war zu erwarten, dass wegen der ähnlichen Molekülstruktur bei annähernd gleichen Molgewichten auch ähnlich hohe Flüchtigkeiten beobachtet werden würden. Des weiteren liess sich keineswegs vorhersehen, dass insbesondere die Esterderivate besser zur Stabilisierung von Lakken geeignet sein würden als die zitierten Alkylprodukte wegen der ähnlichen Polarität. Insgesamt war somit nicht mit anwendungstechnischen Vorteilen gegenüber den zitierten Verbindungen des Standes der Technik zu rechnen. Das im Vergleich zu diesen hervorstehende anwendungstechnische Gesamtbild muss daher als überaus überraschend angesehen werden.

Besonders überraschen muss auch den Fachmann die geringe Flüchtigkeit der neuen Produkte mit $n = 1$. Weiss man doch aus der DE-OS 2 933 732, dass alkylierte Mono-Diazaspirodecane eine recht hohe Flüchtigkeit aufweisen.

Die erfindungsgemässen Verbindungen werden – wie bereits ausgeführt – als Stabilisatoren für Kunststoffe gegen deren Schädigung durch Einwirkung von Sauerstoff, Wärme und Licht verwendet. Beispiele für solche Kunststoffe und auch zusätzlich einsetzbarer Additive sind auf den Seiten 14 bis 19 der DE-OS 3 045 839 genannt.

Besonders geeignet sind die erfindungsgemässen Produkte für die Stabilisierung von Homo- und

Copolymeren des Ethylens, Propylens, Butadiens, von (Meth)Acrylsäurederivaten, besonders deren Estern, und des Styrols, wo man sie im allgemeinen in Mengen von 0,01 bis 5, vorzugsweise 0,05 bis 2,5 und insbesondere 0,1 bis 1,0 Gew.-%, bezogen auf zu stabilisierendes Material, – gegebenenfalls neben anderen stabilisierend wirkenden Stoffen – einsetzt. Für die Stabilisierung von Lacksystemen sind besonders die Produkte geeignet, in denen Y = –O– ist.

Die nachfolgenden Beispiele dienen der weiteren Erläuterung des Erfindungsgegenstandes.

In den folgenden Herstellungsbeispielen sind die Verfahrensprodukte durch Ziffern gekennzeichnet, welche sich auf die Numerierung auf den Seiten 5 bis 7 aufgelisteten Verbindungen beziehen.

Beispiel 1 (Verbindung Nr. 20)

In 400 ml wasserfreiem Xylol werden 109,2 g (0,3 Mol) trockenes

2,2,4,4-Tetramethyl-7-oxa-3,20-diaza-21-oxo-dispiro-[4,5]-heneicosan

vorgelegt. Man fügt 9,0 g (0,3 Mol) 80%iges NaH-Paraffingemisch zu und erhitzt innerhalb einer Stunde auf Rückfluss, worauf bis zur beendeten Wasserstoffentwicklung gerührt wird (weitere 3 Stunden). Dann wird auf 20 °C abgekühlt und eine Lösung aus 50 ml wasserfreiem Xylol und 51,0 g (0,3 Mol) Bromessigsäureethylester zugetropft. Anschliessend wird 8 Stunden unter Rückfluss gerührt, sodann heiss filtriert (38 g NaBr), das Filtrat im Vakuum zur Trockene eingeengt und der Rückstand aus n-Heptan umkristallisiert.

Ausbeute: 119,8 g = 88% d. Th.; Fp. 154 °C.

Beispiele 2 bis 18

Man arbeitet analog Beispiel 1 unter Einsatz des aus der Aufstellung der Verfahrensprodukte auf den Seiten 5 bis 7 jeweils ableitbaren Spirodecans und dem in nachstehender Tabelle verzeichneten Halogenessigsäurederivat.

| Beisp. Nr. | eingesetztes Halogenessigsäurederivat | Verfahrensprodukt Verb.-Nr. | Fp.(° C) |
|---|---|---|---|
| 2 | $BrCH_2COOC_2H_5$ | 19 | 149 |
| 3 | " | 30 | 91 |
| 4 | " | 5 | 74–6 |
| 5 | " | 1 | 83 |
| 6 | " | 2 | 111 |
| 7 | $ClCH_2COOCH_3$ | 10 | 68 |
| 8 | " | 11 | 86 |
| 9 | " | 12 | 73 |
| 10 | " | 13 | 114 |
| 11 | $ClCH_2CONH_2$ | 14 | 85 |
| 12 | " | 17 | 233 |
| 13 | " | 18 | 160 |
| 14 | 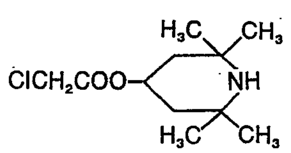 | 3 | 99 |
| 15 | " | 9 | 92 |
| 16 | " | 21 | 135 |
| 17 | $(ClCH_2COOCH_2-)_2$ | 15 | 154 |
| 18 | " | 16 | 172 |

Beispiel 19 (Verbindung Nr. 7)

In 150 g wasserfreiem Mesitylen werden 27,5 g (0,077 Mol) der nach Beispiel 4 hergestellten Verbindung Nr. 5, 4,6 g (0,039 Mol) 1,6-Hexandiol und 0,5 g $LiNH_2$ 15 Stunden unter Rückfluss gerührt, wobei über eine 10-cm-Vigreux-Kolonne 3,5 g Ethanol abdestillieren. Anschliessend wird heiss filtriert und i. Vak. zur Trockene eingeengt. Das zurückbleibende ölige Produkt wird aus Hexan kristallisiert.

Ausbeute: 25,5 g = 89% d. Th.; Fp. 154 °C.

MG: 715 (Theorie 734)

Beispiele 20 bis 31
Arbeitsweise analog Beispiel 19

| Bsp. Nr. | Edukte Alkohol bzw. Amin | Edukt (Ia) = Verb. Nr. | Verfahrensprodukt Verb.-Nr. | Fp(°C) |
|---|---|---|---|---|
| 20 | 2–Ethylhexanol | 20 | 22 | Öl |
| 21 | n–Hexanol–1 | 20 | 23 | ″ |
| 22 | Glycerin | 20 | 24 | Harz |
| 23 | Hexamethylendiamin–1,6 | 5 | 8 | 187 |
| 24 | Hexandiol–1,6 | 20 | 25 | 68 |
| 25 | 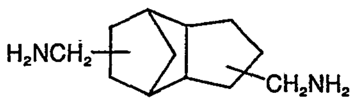 | 1 | 30 | 105 |
| 26 | 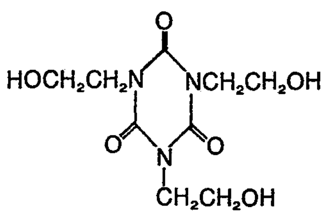 | 1 | 29 | 90 |
| 27 | Glycerin | 2 | 28 | 86 |
| 28 | wie bei Beispiel 25 | 20 | 27 | 100 |
| 29 | Ethylendiamin | 2 | 4 | 248 |
| 30 | Hexamethylendiamin–1,6 | 20 | 26 | 178 |
| 31 | Hexandiol–1,6 | 5 | 7 | 77 |

Beispiel 32

Dieses Beispiel zeigt die Flüchtigkeit der erfindungsgemässen Stabilisatoren im Vergleich zu Produkten des Standes der Technik.

Die Flüchtigkeiten wurden in einer Apparatur zur thermogravimetrischen Analyse bestimmt. Gleiche Mengen (500 mg) der erfindungsgemässen Stabilisatoren und der Vergleichssubstanzen wurden dazu in Stickstoffatmosphäre mit einer Aufheizgeschwindigkeit von 2 K/min bis auf 300 °C aufgeheizt und der Substanzverlust in mg/cm² Probenoberfläche gemessen. Die Ergebnisse zeigt nachfolgende Tabelle:

| Stabilisator gemäss Beispiel | Gewichtsverlust in mg/cm² beim Erreichen von... °C 220 | 260 | 300 | 10 min bei 300 |
|---|---|---|---|---|
| 30 | 0,16 | 0,63 | 1,90 | 3,16 |
| 1 | 0,96 | 4,74 | 19,28 | 32,70 |
| 23 | 0,47 | 0,79 | 3,48 | 5,37 |
| Vergleich[1] | 0,01 | 0,23 | 2,05 | 3,79 |
| Vergleich[2] | 0,79 | 3,63 | 13,27 | 20,22 |
| Vergleich[3] | 2,73 | 6,36 | 30,02 | 52,14 |

[1] Verbindung nach Beispiel 70 der DE-A 2 933 732
[2] Verbindung nach Beispiel 4 der DE-A 2 933 732
[3] Verbindung nach Beispiel 63 der DE-A 2 933 732

Beispiel 33

Eine in einem Laborschnellmischer hergestellte Mischung aus

100 Gew.-T. Polypropylen(®)Hostalen PPU VP 1770 F der Hoechst AG vom Schmelzindex MFI 190/5 = 1,9 g/10 min, bestimmt nach DIN 53 535),

0,2 Gew.-T. Calciumstearat,

0,1 Gew.-T. Pentaerythrityl-tetrakis-3-(3,5-di-tert.-butyl-4-hydroxiphenyl)-propionat und

0,3 Gew.-T. des zu prüfenden Stabilisators

wird zu Granulat verarbeitet. Das derart stabilisierte Material schmilzt man sodann in einem Laborextruder unter den üblichen Verarbeitungsbedingungen auf und verspinnt es über eine Spinnpumpe mit Achtfachspinnkopf zu Monofilamenten. Diese werden anschliessend im Verhältnis 1:3 nachverstreckt und zu Garn von 40 dtex texturiert, welches man zu Prüfgeweben verarbeitet.

Die Gewebeproben werden auf einen gelochten Karton so aufgespannt, dass eine freie Öffnung von ca. 15,5 mm Durchmesser bestehen bleibt und in dieser Form in einer Xenotest-X-1200-Apparatur der Firma Original Hanau Quarzlampen GmbH einer Bestrahlung mit Wechsellicht unterworfen. Hierbei wird die Strahlungsintensität durch UV-Filter (Spezialfilterglas d = 1,7 mm) moduliert und die Prüfmethode nach DIN 53 387 (17 min. Trokkenperiode, 3 min. beregnen, Schwarztafeltemperatur 45 °C, relative Luftfeuchtigkeit während der

Trockenperiode 70 bis 75%) angewandt. In bestimmten Zeitabständen werden die Gewebe zentrisch mit einem Gewicht von 6 mm Durchmesser und einem Druck von 0,1 $N/mm^2$ belastet. Als Versagenstermin gilt das Durchbrechen des Gewichts.

| Stabilisator nach Beispiel Nr. | Belichtungszeit in h |
|---|---|
| 23 | 900[x] |
| 24 | 900[x] |
| Vergleich[1] | 900 |
| Vergleich[2] | 800 |

[1] Verbindung nach Beispiel 70 der DE-A 2 933 732
[2] Verbindung nach Beispiel 4 der DE-A 2 933 732
[x] Gewicht noch nicht durchgebrochen

**Patentansprüche für die Vertragsstaaten BE, CH, DE, FR, GB, IT, LI, NL, SE**

1. Polyalkyldiazaspirodecanylessigsäurederivate der allgemeinen Formel I

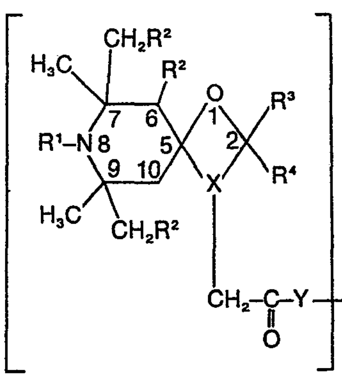

(I),

in welcher

X die Bedeutung einer Gruppe der Formel II oder III hat,

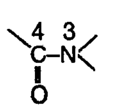 (II), 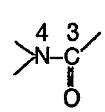 (III)

wobei die Indices 3 und 4 die Ringposition im Diazaspirodecansystem angeben und die freie Valenz des Stickstoffatoms 3 bzw. 4 die Verknüpfung mit dem Essigsäurerest bewirkt,

$R^1$ H, O oder $C_1$- bis $C_{12}$-Alkyl bedeutet,

$R^2$ H oder eine $C_1$- bis $C_5$-Alkylgruppe bedeutet,

$R^3$ und $R^4$ gleich oder verschieden sind und für H, für eine $C_1$- bis $C_{30}$-Alkylgruppe, für eine Phenyl- oder Naphthylgruppe, die durch Chlor oder $C_1$- bis $C_4$-Alkyl substituiert sein kann, oder für eine $C_7$- bis $C_{12}$-Phenylalkylgruppe, die durch $C_1$- bis $C_4$-Alkyl substituiert sein kann, stehen, oder auch

$R^3$ und $R^4$ zusammen mit dem sie bindenden Kohlenstoffatom die Bedeutung einer $C_5$- bis $C_{18}$-Cycloalkylgruppe, die durch bis zu vier $C_1$- bis $C_4$-Alkylgruppen substituiert sein kann, oder einer Gruppe der Formel

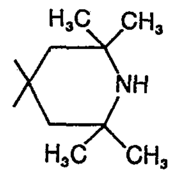

haben,

n für eine ganze Zahl von 1 bis 6 steht, und

$R^5$ bei n = 1, H, $C_1$- bis $C_{18}$-Alkyl, $C_3$- bis $C_{12}$-Alkenyl, $C_7$- bis $C_9$-Phenylalkyl, das durch $C_1$- bis $C_4$-Alkyl substituiert sein kann, oder eine 2,2,6,6-Tetramethxl-4-piperidinylgruppe bedeutet,

bei n = 2, $C_2$- bis $C_{30}$-Alkylen, eine $C_4$- bis $C_8$-Alkenylengruppe, eine $C_2$- bis $C_{12}$-Bis-(propyloxi)-alkylengruppe, oder ein Mono-, Di- oder Trichloralkylen mit 6 bis 18 C-Atomen, das durch bis zu vier Methylgruppen substituiert sein kann, wobei bei dem erstgenannten zwei C-Atome durch N-Atome, welche Propylengruppen tragen können, ersetzt sein können, oder $C_6$- bis $C_{18}$-Arylen, oder $C_7$- bis $C_{18}$-Aralkylen bedeutet,

bei n = 3 die Bedeutung von $C_3$- bis $C_6$-Alkantriyl oder eines Restes der Formel

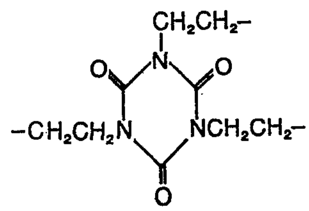

oder eines Di-$C_2$- bis $C_4$-Alkylen-triamino-Restes hat, und

bei n = 4 bis 6 für einen $C_4$- bis $C_{10}$-Alkan-n-yl-Rest, der eine Ethergruppe enthalten kann, oder für einen Tri- bis Penta-$C_2$- bis $C_4$-Alkylen-tetra-bis-hex-amino-Rest steht, und

Y –O– oder $-\overset{R^6}{\overset{|}{N}}-$ bedeutet, wobei $R^6$ eine der unter $R^5$ für n = 1 angegebenen Bedeutungen hat.

2. Verfahren zur Herstellung der Verbindungen nach Anspruch 1, dadurch gekennzeichnet, dass man Azaspirodecane der Formel IV

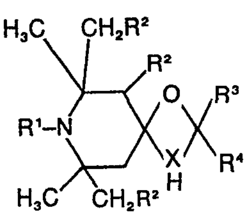

(IV)

in der X und die Reste $R^1$ bis $R^4$ die in Anspruch 1 angegebene Bedeutung haben, durch Umsetzen mit der äquivalenten Menge eines Alkalimetallhydrides, -alkoholats oder -hydroxids in einem inerten organischen Lösemittel bei 50 bis 160 °C in ihre Alkalimetallsalze überführt, worauf diese mit

der äquivalenten Menge einer Halogenverbindung der Formel V

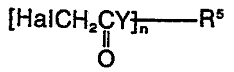

(V)

in der Hal = Cl oder Br und Y, $R^5$ und n die in Anspruch 1 angegebene Bedeutung besitzen, bei 100 bis 200 °C in einem inerten organischen Solvens zur Reaktion gebracht werden.

3. Verfahren nach Anspruch 2, dadurch gekennzeichnet, dass man das Alkalisalz der Verbindung IV zunächst mit einem Halogenessigsäuremethyl- oder -ethylester umsetzt, wobei eine Verbindung der Formel I mit n = 1, Y = –O– und $R^5 = CH_3$ bzw. $C_2H_5$ erhalten wird, worauf man diese in einem inerten organischen Solvens bei 100 bis 200 °C in Gegenwart alkalischer Katalysatoren mit der äquivalenten Menge einer Verbindung der Formel VI

$$R^5\text{–}(Y\text{–}H)_n \qquad (VI)$$

in der $R^5$, Y und n die in Anspruch 1 genannten Bedeutungen haben, reagieren lässt.

4. Verwendung der Verbindungen nach Anspruch 1 zum Stabilisieren von synthetischen Polymeren.

5. Verwendung nach Anspruch 4, dadurch gekennzeichnet, dass das Polymere ein Polyolefin, ein Polyacrylat, ein Polymethacrylat oder ein Homo- oder Copolymeres von Styrol ist.

6. Verwendung der Verbindungen nach Anspruch 1 mit Y = –O– zum Stabilisieren von Lakken.

7. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

8. Gegen UV-Zersetzung stabilisierte, synthetische Polymere, in denen 0,01 bis 5 Gewichtsteile, bezogen auf Polymeres, eines Stabilisators nach Anspruch 1 enthalten sind.

**Patentansprüche für den Vertragsstaat AT**

1. Verfahren zur Herstellung von Polyalkyldiazaspirodecanylessigsäurederivaten der allgemeinen Formel I

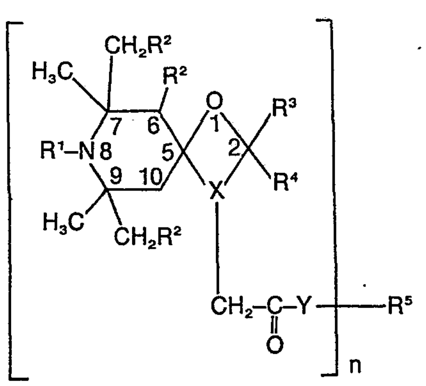

(I),

in welcher

X die Bedeutung einer Gruppe der Formel II oder III hat,

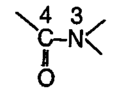 (II), 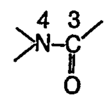 (III)

wobei die Indices 3 und 4 die Ringposition im Diazaspirodecansystem angeben und die freie Valenz des Stickstoffatoms 3 bzw. 4 die Verknüpfung mit dem Essigsäurerest bewirkt,

$R^1$ H, O oder $C_1$- bis $C_{12}$-Alkyl bedeutet,

$R^2$ H oder eine $C_1$- bis $C_5$-Alkylgruppe bedeutet,

$R^3$ und $R^4$ gleich oder verschieden sind und für H, für eine $C_1$- bis $C_{30}$-Alkylgruppe, für eine Phenyl- oder Naphthylgruppe, die durch Chlor oder $C_1$- bis $C_4$-Alkyl substituiert sein kann, oder für eine $C_7$- bis $C_{12}$-Phenylalkylgruppe, die durch $C_1$- bis $C_4$-Alkyl substituiert sein kann, stehen, oder auch

$R^3$ und $R^4$ zusammen mit dem sie bindenden Kohlenstoffatom die Bedeutung einer $C_5$- bis $C_{18}$-Cycloalkylgruppe, die durch bis zu vier $C_1$- bis $C_4$-Alkylgruppen substituiert sein kann, oder einer Gruppe der Formel

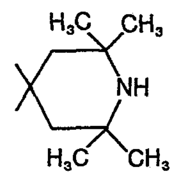

haben,

n für eine ganze Zahl von 1 bis 6 steht, und

$R^5$ bei n = 1, H, $C_1$- bis $C_{18}$-Alkyl, $C_3$- bis $C_{12}$-Alkenyl, $C_7$- bis $C_9$-Phenylalkyl, das durch $C_1$- bis $C_4$-Alkyl substituiert sein kann, oder eine 2,2,6,6-Tetramethyl-4-piperidinylgruppe bedeutet,

bei n = 2, $C_2$- bis $C_{30}$-Alkylen, eine $C_4$- bis $C_8$-Alkenylengruppe, eine $C_2$- bis $C_{12}$-Bis-(propyloxi)-alkylengruppe, oder ein Mono-, Di- oder Tricycloalkylen mit 6 bis 18 C-Atomen, das durch bis zu vier Methylgruppen substituiert sein kann, wobei bei dem erstgenannten zwei C-Atome durch N-Atome, welche Propylengruppen tragen können, ersetzt sein können, oder $C_6$- bis $C_{18}$-Arylen, oder $C_7$- bis $C_{18}$-Aralkylen bedeutet,

bei n = 3 die Bedeutung von $C_3$- bis $C_6$-Alkentriyl oder eines Restes der Formel

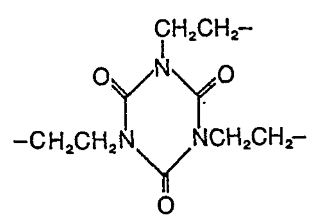

oder eines Di-$C_2$- bis $C_4$-Alkylen-triamino-Restes hat, und

bei n = 4 bis 6 für einen $C_4$- bis $C_{10}$-Alkan-n-yl-Rest, der eine Ethergruppe enthalten kann, oder für einen Tri- bis Penta-$C_2$- bis $C_4$-Alkylen-tetra-bis-hex-amino-Rest steht, und

Y –O– oder $-\overset{R^6}{\overset{|}{N}}-$ bedeutet, wobei $R^6$ eine der

unter $R^5$ für n = 1 angegebenen Bedeutungen hat, dadurch gekennzeichnet, dass man Azaspirodecane der Formel IV

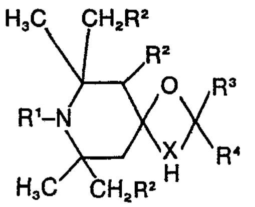

(IV)

in der X und die Reste $R^1$ bis $R^4$ die oben angegebene Bedeutung haben, durch Umsetzen mit der äquivalenten Menge eines Alkalimetallhydrids, -alkoholats oder -hydroxids in einem inerten organischen Lösemittel bei 50 bis 160 °C in ihre Alkalimetallsalze überführt, worauf diese mit der äquivalenten Menge einer Halogenverbindung der Formel V

$$[HalCH_2\underset{\underset{O}{\|}}{C}Y]_n—R^5 \quad (V)$$

in der Hal = Cl oder Br und Y, $R^5$ und n die oben angegebene Bedeutung besitzen, bei 100 bis 200 °C in einem inerten organischen Solvens zur Reaktion gebracht werden.

2. Verfahren nach Anspruch 1, dadurch gekennzeichnet, dass man das Alkalisalz der Verbindung IV zunächst mit einem Halogenessigsäuremethyl- oder -ethylester umsetzt, wobei eine Verbindung der Formel I mit n = 1, Y = –O– und $R^5 = CH_3$ bzw. $C_2H_5$ erhalten wird, worauf man diese in einem inerten organischen Solvens bei 100 bis 200 °C in Gegenwart alkalischer Katalysatoren mit der äquivalenten Menge einer Verbindung der Formel IV

$$R^5–(Y–H)_n \quad (VI)$$

in der $R^5$, Y und n die oben genannten Bedeutungen haben, reagieren lässt.

3. Verwendung der nach Anspruch 1 erhaltenen Verbindungen zum Stabilisieren von synthetischen Polymeren.

4. Verwendung nach Anspruch 3, dadurch gekennzeichnet, dass das Polymere ein Polyolefin, ein Polyacrylat, ein Polymethacrylat oder ein Homo- oder Copolymeres von Styrol ist.

5. Verwendung der nach Anspruch 1 erhaltenen Verbindungen mit Y = –O– zum Stabilisieren von Lacken.

6. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, eines nach Anspruch 1 erhaltenen Stabilisators zusetzt.

7. Gegen UV-Zersetzung stabilisierte, synthetische Polymere, in denen 0,01 bis 5 Gewichtsteile, bezogen auf Polymeres, eines nach Anspruch 1 hergestellten Stabilisators enthalten sind.

**Claims for the contracting States: BE, CH, DE, FR, GB, IT, LI, NL, SE**

1. A polyalkyldiazaspirodecanylacetic acid derivative of the general formula I

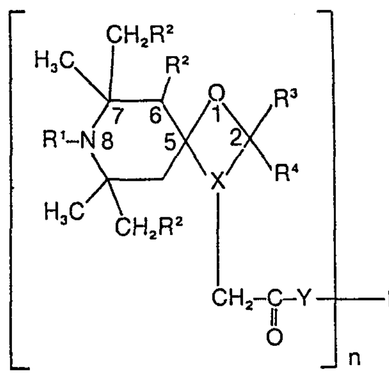

(I),

in which X denotes a group of the formula II or III

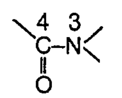

(II),

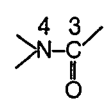

(III)

in which the indices 3 and 4 indicate the ring position in the diazaspirodecane system, and the free valency of the nitrogen atom 3 or 4 effects the linkage with the acetic acid radical, $R^1$ is H, O or $C_1$ to $C_{12}$ alkyl, $R^2$ is H or a $C_1$ to $C_5$ alkyl group, $R^3$ and $R^4$ are identical or different and represent H, a $C_1$ to $C_{30}$ alkyl group, a phenyl or naphthyl group which can be substituted by chlorine or $C_1$ to $C_4$ alkyl, or a $C_7$ to $C_{12}$ phenylalkyl group which can be substituted by $C_1$ to $C_4$ alkyl, or $R^3$ and $R^4$, together with the carbon atom linking them, also denote a $C_5$ to $C_{18}$ cycloalkyl group which can be substituted by up to four $C_1$ to $C_4$ alkyl groups, or a group of the formula

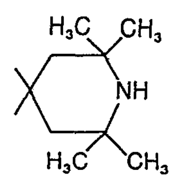

n represents an integer from 1 to 6 and, if n = 1, $R^5$ denotes H, $C_1$ to $C_{18}$ alkyl, $C_3$ to $C_{12}$ alkenyl, $C_7$ to $C_9$ phenylalkyl which can be substituted by $C_1$ to $C_4$ alkyl, or a 2,2,6,6,-tetramethyl-4-piperidinyl group, if n = 2, $R^5$ is $C_2$ to $C_{30}$ alkylene, a $C_4$ to $C_8$ alkenylene group, a $C_2$ to $C_{12}$ bis-(propyloxy)-alkylene group or a monocycloalkylene, dicycloalkylene or tricycloalkylene which has 6 to 18 carbon atoms and which can be substituted by up to four methyl groups, it being possible, in the case first mentioned, for two carbon atoms to be replaced by nitrogen atoms which can carry propylene groups, or $R^5$ is $C_6$ to $C_{18}$ arylene or $C_7$ to $C_{18}$ aralkylene, if n = 3, $R^5$ denotes $C_3$ to $C_6$ alkanetriyl or a radical of the formula

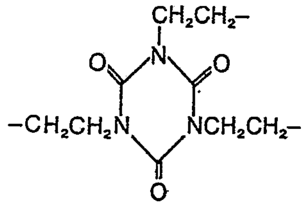

or a di-$C_2$-alkylenetriamino to di-$C_4$-alkylenetriamino radical and, if n = 4 to 6, $R^5$ represents a $C_4$ to $C_{10}$ alkan-n-yl radical which can contain an ether group, or represents a tri-$C_2$ to $C_4$ alkylenetetra-bis-hexamino to penta-$C_2$ to $C_4$-alkylenetetra-bis-hexamino radical, and Y is –O– or

$R^6$
|
–N– in which $R^6$ has one of the meanings indicated under $R^5$ in the case where n = 1.

2. A process for the preparation of a compound as claimed in claim 1, which comprises converting an azaspirodecane of the formula IV

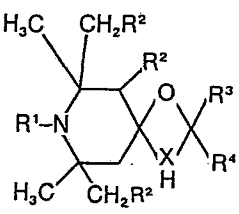
(IV)

in which X and the radicals $R^1$ to $R^4$ have the meaning indicated in claim 1, into an alkaly metal salt thereof by reacting it with an equivalent quantity of an alkali metal hybride, alcoholate or hydroxide in an inert organic solvent at 50 to 160 °C, and then reacting this salt, at 100 to 200 °C, in an inert organic solvent, with an equivalent quantity of a halogen compound of the formula V

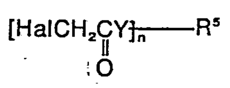
(V)

in which HAl = Cl or Br and Y, $R^5$ and n have the meaning indicated in claim 1.

3. The process as claimed in claim 2, wherein the alkali metal salt of the compound IV is first reacted with a methyl or ethyl halogenoacetate, whereby a compound of the formula I in which n = 1, Y = –O– and R = $CH_3$ or $C_2H_5$ is obtained, and this compound is then reacted, in an inert organic solvent at 100 to 200 °C, in the presence of alkaline catalysts, with an equivalent quantity of a compound of the formual VI

$$R^5\text{–}(Y\text{–}H)_n \qquad (VI)$$

in which $R^5$, Y and n have the meanings mentioned in claim 1.

4. The use of a compound as claimed in claim 1 for stabilizing synthetic polymers.

5. Use as claimed in claim 4, wherein the polymer is a polyolefine, a polyacrylate, a polymethacrylate or a homopolymer or copolymer of styrene.

6. The use of a compound as claimed in claim 1 in which Y = –O– for stabilizing lacquers.

7. A process for stabilizing synthetic polymers against the harmful effects of light, which comprises adding to the polymers, if appropriate as well as hitherto known substances having a stabilizing action, 0.01 to 5 parts by weight, relative to the polymer, of a stabilizer as claimed in claim 1.

8. A synthetic polymer which has been stabilized against decomposition by UV and which contains 0.01 to 5 parts by weight, relative to the polymer, of a stabilizer as claimed in claim 1.

**Claims for the contracting State: AT**

1. A process for the manufacture of a polyalkyldiazaspirodecanylacetic acid derivative of the general formula I

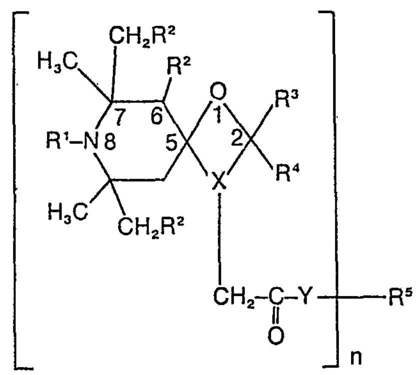
(I),

in which X denotes a group of the formula II or III

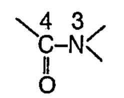
(II),
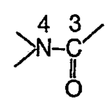
(III)

in which the indices 3 and 4 indicate the ring position in the diazaspirodecane system, and the free valency of the nitrogen atom 3 or 4 effects the linkage with the acetic acid radical, $R^1$ is H, O or $C_1$ to $C_{12}$ alkyl, $R^2$ is H or a $C_1$ to $C_5$ alkyl group, $R^3$ and $R^4$ are identical or different and represent H, a $C_1$ to $C_{30}$ alkyl group, a phenyl or naphthyl group which can be substituted by chlorine or $C_1$ to $C_4$ alkyl, or a $C_7$ to $C_{12}$ phenylalkyl group which can be substituted by $C_1$ to $C_4$ alkyl, or $R^3$ and $R^4$, together with the carbon atom linking them, also denote a $C_5$ to $C_{18}$ cycloalkyl group which can be substituted by up to four $C_1$ to $C_4$ alkyl groups, or a group of the formula

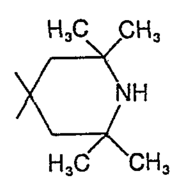

n represents an integer from 1 to 6 and, if n = 1, $R^5$ denotes H, $C_1$ to $C_{12}$ alkenyl, $C_7$ to $C_9$ phenylalkyl which can be substituted by $C_1$ to $C_4$ alkyl, or a 2,2,6,6-tetramethyl-4-piperidinyl group, if n = 2, $R^5$

is $C_2$ to $C_{30}$ alkylene, a $C_4$ to $C_8$ alkenylene group, a $C_2$ to $C_{12}$ bis-(propyloxy)-alkylene group or a monocycloalkylene, dicycloalkylene or tricycloalkylene which has 6 to 18 carbon atoms and which can be substituted by up to four methyl groups, it being possible, in the case first mentioned, for two carbon atoms to be replaced by nitrogen atoms which can carry propylene groups, or $R^5$ is $C_6$ to $C_{18}$ arylene or $C_7$ to $C_{18}$ aralkylene, if n = 3, $R^5$ denotes $C_3$ to $C_6$ alkanetriyl or a radical of the formula

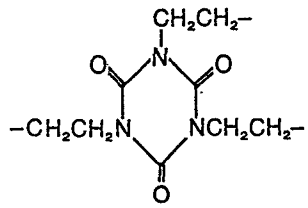

or a di-$C_2$-alkylentrioamino to di-$C_4$-alkylenetriamino radical and, if n = 4 to 6, $R^5$ represents a $C_4$ to $C_{10}$ alkan-n-yl radical which can contain an ether group, or represents a tri-$C_2$ to $C_4$ alkylenetetra-bis-hexamino to penta-$C_2$ to $C_4$-alkylenetetra-bis-hexamino radical, and Y is –O– or 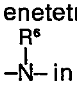 in which $R^6$ has one of the meanings indicated under $R^5$ in the case where n = 1, which comprises converting an azaspirodecane of the formula IV

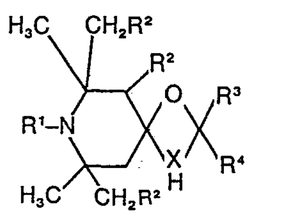

(IV)

in which X and the radicals $R^1$ to $R^4$ have the meaning indicated in claim 1, into an alkali metal salt thereof by reacting it with an equivalent quantity of an alkali metal hydride, alcoholate or hydroxide in an inert organic solvent at 50 to 160 °C, and then reacting this salt, at 100 to 200 °C in an inert organic solvent, with an equivalent quantity of a halogen compound of the formula V

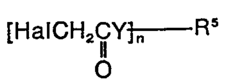

(V)

in which Hal = Cl or Br and Y, $R^5$ and n have the meaning indicated in claim 1.

2. The process as claimed in claim 1, wherein the alkali metal salt of the compound IV is first reacted with a methyl or ethyl halogenoacetate, whereby a compound of the formula I in which n = 1, Y = –O– and R = $CH_3$ or $C_2H_5$ is obtained, and this compound is then reacted, in an inert organic solvent at 100 to 200 °C, in the presence of alkaline catalysts, with an equivalent quantity of a compound of the formula VI

$$R^5\text{–}(Y\text{–}H)_n \qquad (VI)$$

in which $R^5$, Y and n have the meanings mentioned in claim 1.

3. The use of a compound obtained according to claim 1 for stabilizing synthetic polymers.

4. Use as claimed in claim 3, wherein the polymer is a polyolefine, a polyacrylate, a polymethacrylate or a homopolymer or copolymer of styrene.

5. The use of a compound as obtained according to claim 1 in which Y = –O– for stabilizing lacquers.

6. A process for stabilizing synthetic polymers against the harmful effects of light, which comprises adding to the polymers, if appropriate as well as hitherto known substances having a stabilizing action, 0.01 to 5 parts by weight, relative to the polymer, of a stabilizer as obtained according to claim 1.

7. A synthetic polymer which has been stabilized against decomposition by UV and which contains 0.01 to 5 parts by weight, relative to the polymer, of a stabilizer as obtained according to claim 1.

**Revendications pour les Etats contractants: BE, CH, DE, FR, GB, IT, LI, NL, SE**

1. Dérivé d'acides (polyalkyl-diazaspirodécyl)-acétiques répondant à la formule générale I:

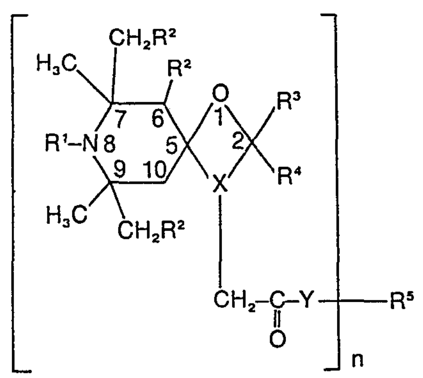

(I),

dans laquelle:

X représente un radical répondant à l'une des formules II et III:

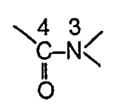

(II),

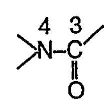

(III)

dans lesquelles les indices 3 et 4 indiquent les positions qu'occupent les atomes correspondants dans le système du diazaspirodécane et la valence libre de l'atome d'azote en 3 ou 4 assure la liaison avec le radical de l'acide acétique,

$R^1$ représente H, O ou un alkyle en $C_1$–$C_{12}$,

$R^2$ représente H ou un alkyle en $C_1$–$C_5$,

$R^3$ et $R^4$ représentent chacun, indépendamment l'un de l'autre, H, un alkyle en $C_1$–$C_{30}$, un radical phényle ou naphthyle éventuellement porteur d'un atome de chlore ou d'un alkyle en $C_1$–$C_4$, ou un radical phénylalkyle en $C_7$–$C_{12}$, éventuellement porteur d'un alkyle en $C_1$–$C_4$, ou encore

$R^3$ et $R^4$ forment ensemble, et avec l'atome de carbone auquel ils sont liés, un radical cycloalkyle en $C_5$–$C_{18}$ éventuellement porteur d'1 à 4 radicaux alkyles en $C_1$–$C_4$, ou un radical de formule:

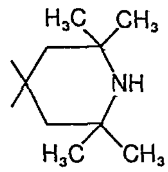

n représente un nombre entier de 1 à 6,

$R^5$ représente:

– dans le cas où n est égal à 1, l'hydrogène, un alkyle en $C_1$–$C_{18}$, un alcényle en $C_3$–$C_{12}$, un phénylalkyle en $C_7$–$C_9$ éventuellement porteur d'un alkyle en $C_1$–$C_4$, ou un radical tétraméthyl-2,2,6,6, pipéridyle-4,

– dans le cas où n est égal à 2, un alkylène en $C_2$–$C_{30}$, un alcénylène en $C_4$–$C_8$, un bis-(propyloxy)-alkylène en $C_2$–$C_{12}$, un mono-, di- ou tricycloalkylène en $C_6$–$C_{18}$, éventuellement porteur de 1 à 4 méthyles, deux atomes de carbone du premier cité pouvant être remplacé par des atomes d'azote éventuellement porteurs de radicaux propylènes, un arylène en $C_{6-18}$ ou un aralkylène en $C_7$–$C_{18}$,

– dans le cas où n est égal à 3, un alcane-triyle en $C_3$–$C_6$, un radical de formule:

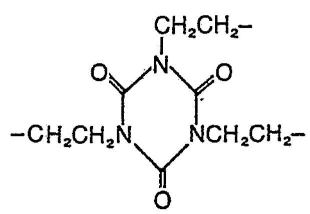

ou un radical di-alkylène-triamino dont les alkylènes contiennent chacun de 2 à 4 atomes de carbone, et

– dans le cas où n est un nombre de 4 à 6, un radical alcane-n-yle en $C_4$–$C_{10}$, qui peut contenir un radical éther, ou un radical tri- à penta-alkylène-tétra- à hexaamino dont les alkylènes contiennent chacun de 2 à 4 atomes de carbone, et

Y représente –O– ou 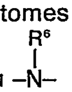

où $R^6$ a l'une des significations qui ont été données à propos de $R^5$ dans le cas où n était égal à 1.

2. Procédé de préparation des composés selon la revendication 1, procédé caractérisé en ce qu'on transforme des azaspirodécanes répondant à la formule IV:

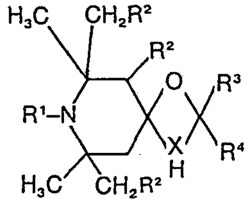

(IV)

dans laquelle X et les symboles $R^1$ à $R^4$ ont les significations données à la revendication 1, par réaction avec la quantité équivalente d'un hydrure, d'un alcoolate ou d'un hydroxyde de métal alcalin, dans un solvant organique inerte et à une température de 50 à 160 °C, en leurs sels de métaux alcalines, puis on fait réagir ceux-ci avec la quantité équivalente d'un composé halogéné répondant à la formule V:

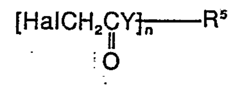

(V)

dans laquelle Hal représente Cl ou Br tandis que Y, $R^5$ et n ont les significations données à la revendication 1, à une température de 100 à 200 °C, dans un solvant organique inerte.

3. Procédé selon la revendication 2 caractérisé en ce qu'on fait réagir le sel de métal alcalin du composé IV d'abord avec un halogéno-acétate de méthyle ou d'éthyle, réaction qui conduit à un composé de formule I dans lequel n est égal à 1, Y représente –O– et $R^5$ représente $-CH_3$ ou $-C_2H_5$, puis on fait réagir celui-ci, dans un solvant organique inerte, à une température de 100 à 200 °C et en présence d'un catalyseur alcalin, avec la quantité équivalente d'un composé répondant à la formule VI:

$$R^5-(Y-H)_n \qquad (VI)$$

dans laquelle $R^5$, R et n ont les significations données à la revendication 1.

4. Application des composés selon la revendication 1 pour la stabilisation de polymères synthétiques.

5. Application selon la revendication 4 caractérisée en ce que le polymère est une polyoléfine, un polyacrylate, un polyméthacrylate ou un homopolymère ou copolymère du styrène.

6. Application des composés selon la revendication 1 dans lesquels Y représente –O– pour la stabilisation de peintures et de vernis.

7. Procédé de stabilisation de poylmères synthétiques contre les effets destructeurs de la lumière, procédé caractérisé en ce qu'on ajoute aux polymères, éventuellement en plus de substances stabilisantes déjà connues, de 0.01 à 5 parties en poids, par rapport aux polymères, d'un stabilisant selon la revendication 1.

8. Polymères synthétiques stabilisés contre la décomposition par les rayons ultra-violets, polymères qui contiennent de 0,01 à 5 parties en poids, par rapport à eux-mêmes, d'un stabilisant selon la revendication 1.

**Revendications pour l'Etat contractant: AT**

1. Procédé de préparation de dérivés d'acides (polyalkyl-diazaspirodécyl)-acétiques répondant à la formule générale I:

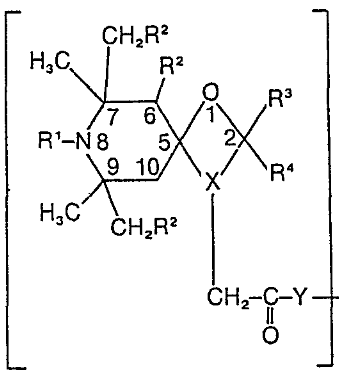

(I),

dans laquelle:

X représente un radical répondant à l'une des formules II et III:

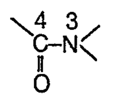 (II), 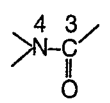 (III)

dans lesquelles les indices 3 et 4 indiquent les positions qu'occupent les atomes correspondants dans le système du diazaspirodécane et la valence libre de l'atome d'azote en 3 ou en 4 assure la liaison avec le radical de l'acide acétique,

$R^1$ représente H, O ou un alkyle en $C_1$–$C_{12}$,

$R^2$ représente H ou un alkyle en $C_1$–$C_5$,

$R^3$ et $R^4$ représentent chacun, indépendamment l'un de l'autre, H, un alkyle en $C_1$–$C_{30}$, un radical phényle ou naphthyle éventuellement porteur d'un atome de chlore ou d'un alkyle en $C_1$–$C_4$, ou un radical phénylalkyle en $C_7$–$C_{12}$, éventuellement porteur d'un alkyle en $C_1$–$C_4$, ou encore

$R^3$ et $R^4$ forment ensemble, et avec l'atome de carbone auquel ils sont liés, un radical cycloalkyle en $C_5$–$C_{18}$ éventuellement porteur d'1 à 4 radicaux alkyles en $C_1$–$C_4$, ou un radical de formule:

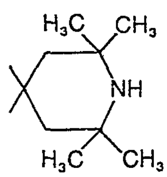

n représente un nombre entier de 1 à 6,

$R^5$ représente:

– dans le cas où n est égal à 1, l'hydrogène, un alkyle en $C_1$–$C_{18}$, un alcényle en $C_3$–$C_{12}$, un phénylalkyle en $C_7$–$C_9$ éventuellement porteur d'un alkyle en $C_1$–$C_4$, ou un radical tétraméthyl-2,2,6,6 pipéridyle-4,

– dans le cas où n est égal à 2, un alkylène en $C_2$–$C_{30}$, un alcénylène en $C_4$–$C_8$, un bis-(propyloxy)-alkylène en $C_2$–$C_{12}$, un mono-, di- ou tricycloalkylène en $C_6$–$C_{18}$, éventuellement porteur de 1 à 4 radicaux méthyles, deux atomes de carbone du premier cité être remplacés par des atomes d'azote éventuellement porteurs de radicaux propylènes, un arylène en $C_6$–$C_{18}$ ou un aralkylène en $C_7$–$C_{18}$,

– dans le où n est égal à 3, un alcane-triyle en $C_3$–$C_6$. un radical de formule:

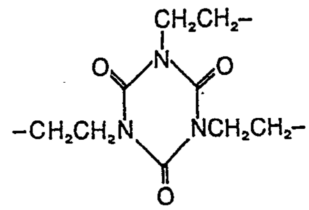

ou un radical di-alkylène-triamino dont les alkylènes contiennent chacun de 2 à 4 atomes de carbone, et

– dans le cas où n est un nombre de 4 à 6, un radical alcane-n-yle en $C_4$–$C_{10}$, qui peut contenir un radical éther, ou un radical tri- à penta-alkylène-tétra- à hexy-amino dont les alkylènes contiennent chacun de 2 à 4 atomes de carbone, et

Y représente –O– ou $-\overset{R^6}{\overset{|}{N}}-$ où $R^6$ a l'une des significations qui ont été données à propos de $R^5$ dans le cas où n était égal à 1, procédé caractérisé en ce qu'on transforme des azaspirodécanes répondant à la formule IV:

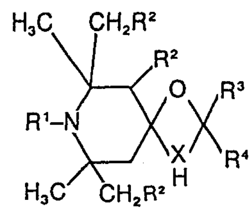

(IV)

dans laquelle X et les symboles $R^1$ à $R^4$ ont les significations précédemment données, par réaction avec la quantité équivalente d'un hydrure, d'un alcoolate ou d'un hydroxyde de métal alcalin, dans un solvant organique inerte et à une température de 50 à 160 °C, en leurs sels de métaux alcalins, puis on fait réagir ceux-ci avec la quantité équivalente d'un composé halogéné répondant à la formule V:

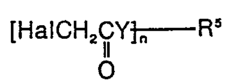

(V)

dans laquelle Hal représente Cl ou Br et Y, $R^5$ et n ont les significations précédemment données, à une température de 100 à 200 °C et dans un solvant organique inerte.

2. Procédé selon la revendication 1 caractérisé en ce qu'on fait réagir le sel de métal alcalin du composé (IV) d'abord avec un halogéno-acétate de méthyle ou d'éthyle, réaction qui conduit à un composé de formule I dans lequel n est égal à 1, Y représente –O– et $R^5$ représente $-CH_3$ ou $-C_2H_5$, après quoi on fait réagir celui-ci, dans un solvant organique inerte, à une température de 100 à 200 °C et en présence d'un catalyseur alcalin, avec la quantité équivalente d'un composé répondant à la formule VI:

$$R^5-(Y-H)_n \quad \text{(VI)}$$

dans laquelle $R^5$, Y et n ont les significations indiquées ci-dessus.

3. Application des composés obtenus selon la revendication 1 pour la stabilisation de polymères synthétiques.

4. Application selon la revendication 3 caractérisée en ce que le polymère est une polyoléfine, un polyacrylate, un polyméthacrylate ou un homopolymère ou copolymère du styrène.

5. Application des composés obtenus selon la revendication 1 dans lesquels Y représente –O– pour la stabilisation de peintures et de vernis.

6. Procédé pour stabiliser des polymères synthétiques contre les effets destructeurs de la lumière, procédé caractérisé en ce qu'on ajoute aux polymères, éventuellement en plus d'autres substances stabilisantes déjà connues, de 0,01 à 5 parties en poids, par rapport aux polymères, d'un stabilisant obtenu selon la revendication 1.

7. Polymères synthétiques stabilisés contre la décomposition due aux ultraviolets, polymères qui contiennent de 0,01 à 5 parties en poids, par rapport à eux-mêmes, d'un stabilisant qui a été préparé selon la revendication 1.